# EUROPEAN PATENT SPECIFICATION

(11) **EP 0 648 494 B1**
(45) Date of publication and mention of the grant of the patent: **25.11.1998**
(21) Application number: 94307117.5
(22) Date of filing: 29.09.1994
(51) Int. Cl.: A61K 31/71, A61K 9/08, A61K 47/00, A61K 47/18

(54) **Rapamycin formulations for oral administration**
Rapamycin-Zubereitungen zur oralen Verabreichung
Formulations de rapamycine pour l'administration orale

(30) Priority: 30.09.1993 US 129523; 30.09.1993 US 129529
(43) Date of publication of application: 19.04.1995
(73) Proprietor: AMERICAN HOME PRODUCTS CORPORATION, Madison, New Jersey 07940-0874 (US)
(72) Inventor: Waranis, Robert Paul, Clinton County, New York (US); Leonard, Thomas Waymond, Wilmington, North Carolina, 28403 (US); Harrison, Maureen Murphy, St Albans Vermont 05478 (US); Ofslager, Christian Luther, New York (US)
(74) Representative: Wileman, David Francis, Dr.

(56) References cited:
- EP-A- 0 041 795
- EP-A- 0 401 747
- WO-A-92/14737
- TRANSPLANTATION, vol.51, no.1, 1991 pages 22 - 26 STANISLAW M. STEPKOWSKI ET AL. 'Rapamycin, a potent immunosuppressive drug for vascularized heart, kidney and small bowel transplantation in the rat'

## Description

This invention relates to formulations containing rapamycin, or pharmaceutically acceptable salts of rapamycin, which are useful in oral administrations for inducing immunosuppression and for treating transplantation rejection, host vs. graft disease, autoimmune diseases, diseases of inflammation, solid tumors, fungal infections, adult T-cell leukemia/lymphomas and hyperproliferative vascular disorders. This invention also relates to processes for preparing such compositions.

Rapamycin is a macrolide antibiotic produced by *Streptomyces hygroscopicus* which was discovered first for its properties as an antifungal agent. It adversely affects the growth of fungi such as *Candida albicans* and *Microsporum gypseum*. Rapamycin, its preparation and its antibiotic activity were described in U.S. Patent No. 3,929,992, issued December 30, 1975 to Surendra Sehgal et al. In 1977 Martel, R. R. et al. reported on immunosuppressive properties of rapamycin against experimental allergic encephalitis and adjuvant arthritis in the Canadian Journal of Physiological Pharmacology, 55, 48-51 (1977). In 1989, Calne, R. Y. et al. in Lancet, 1989, no. 2, p. 227 and Morris, R. E. and Meiser, B. M. in Medicinal Science Research, 1989, No. 17, P. 609-10, separately reported on the effectiveness of rapamycin in inhibiting rejection *in vivo* in allograft transplantation. Numerous articles have followed describing the immunosuppressive and rejection inhibiting properties of rapamycin, and clinical investigations have begun for the use of rapamycin in inhibiting rejection in transplantation in man.

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R. Y. Calne et al., Lancet 1183 (1978); and U.S. Patent 5,100,899].

Rapamycin has also been shown to be useful in preventing or treating systemic lupus erythematosus [U.S. Patent 5,078,999], pulmonary inflammation [U.S. Patent 5,080,899], insulin dependent diabetes mellitus [Fifth Int. Conf. Inflamm. Res. Assoc. 121 (Abstract), (1990)], and smooth muscle cell proliferation and intimal thickening following vascular injury [Morris, R. J. Heart Lung Transplant 11 (pt. 2): 197 (1992)].

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42- positions. U.S. Patent 5,118,678 discloses carbamates of rapamycin that are useful as immunosuppressive, anti-inflammatory, antifungal, and antitumor agents. U. S. Patent 5,100,883 discloses fluorinated esters of rapamycin. U. S. Patent 5,118,677 discloses amide esters of rapamycin. U. S. Patent 5,130,307 discloses aminoesters of rapamycin. U. S. Patent 5,117,203 discloses sulfonates and sulfamates of rapamycin. U. S. Patent 5,194,447 discloses sulfonylcarbamates of rapamycin.

U.S. Patent No. 5,100,899 (Calne) discloses methods of inhibiting transplant rejection in mammals using rapamycin and derivatives and prodrugs thereof. Other chemotherapeutic agents listed for use with rapamycin are azathioprine, corticosteroids, cyclosporin (and cyclosporin A), and FK-506, or any combination thereof.

EP-A-401747 teaches the preparation of dosage forms of rapamycin in general terms specifically exemplifying a suspension of rapamycin with olive oil as a carrier.

S.M. Stepkowski et al in Transplantation, Vol 51, No 1, 1991 pps 22-26 discloses a specific formulation of rapamycin for administration to the artery of the graft or the lumber vein of the recipient.

WO 92/14737 teaches the preparation of oral liquid preparations of 29-desmethylrapamycin in general terms listing many possible ingredients, one of which is lecithin.

The primary immunosuppressive agent presently used for inhibiting rejection in the allograft transplantation of organs in man is cyclosporine (Sandimmune®). Cyclosporine is a cyclic polypeptide consisting of 11 amino acids. The intravenous injectable formulation of Sandimmune® (IV) is a sterile ampul containing, per ml, 50 mg of cyclosporine, 650 mg of Cremophor® EL and alcohol Ph Helv. (32.9% by volume) (under nitrogen). For administration this mixture is diluted further with 0.9 % Sodium Chloride Injection or 5% Dextrose Injection before use. (Physicians' Desk Reference, 45th ed., 1991, pp. 1962-64, Medical Economics Company, Inc.) The macrolide molecule designated FK506, which has certain structural similarities to rapamycin, is also currently undergoing clinical investigation for inhibiting rejection in allograft organ transplantation in man. FK506 is isolated from *Streptomyces tsuskubaensis* and is described in U.S. Patent No. 4,894,366 to Okuhara et al., issued January 16, 1990 R. Venkataramanan et al., in Transplantation Proceedings, 22, No. 1, Suppl., 1 pp 52-56 (February 1990), report that the intravenous injectable formulation of FK506 is provided as a 10 mg/ml solution of FK506 in polyoxyethylated castor oil (HCO-60, a surfactant) and alcohol. The intravenous preparation must be diluted with saline or dextrose and administered as an infusion for 1 to 2 hours.

The Physicians' Desk Reference (45th ed., 1991, p. 2119, Medical Economics Company, Inc.) lists cyclosporine under the Sandimmune® tradename as available in 25 mg and 100 mg strength capsules and as an oral solution in 50 ml bottles. The 25 mg capsules contain 25 mg cyclosporine, USP, and alcohol, USP dehydrated, at a maximum of 12.7% by volume. The 100 mg capsules contain cyclosporine, USP, 100 mg and alcohol, USP dehydrated, at a maximum 12.7% by volume. Inactive ingredients in the oral capsules are corn oil, gelatin, glycerol, Labrafil M 2125 CS (polyoxyethylated glycolysed glycerides), red iron oxide, sorbitol, titanium dioxide, and other ingredients. The oral solution is available in 50 mg bottles containing cyclosporine, USP, 100 mg and Ph. Helv. alcohol at 12.5% by volume dissolved in olive oil, Ph. Helv./Labrafil M 1944 CS (polyoxyethylated oleic glycerides) vehicle which must be diluted further with milk, chocolate milk or orange juice before oral administration.

Azathioprine (available from Burroughs Wellcome Co., Research Triangle Park, N.C., under the tradename Imuran®) is another orally administered immunosuppressive agent prescribed alone or in conjunction with other immunosuppressive agents. The Physicians' Desk Reference (45th ed., 1991, pp. 785-787, Medical Economics Company, Inc.) lists azathioprine as 6-[1-methyl-4-nitroimidazol-5-yl)thio]purine, which is provided for oral administration in scored tablets containing 50 mg azathioprine and the inactive ingredients lactose, magnesium stearate, potato starch, povidone, and stearic acid.

Methods of drug delivery are designed to deliver an acceptable dosage of the medication to the patient. In the case of oral formulations, it is highly desirable to provide a dosage form which meets this criteria and which can be effectively administered, preferably self-administered, in either clinical or non-clinical situations. The present invention concerns formulations useful in the oral administration of rapamycin. Rapamycin has been shown to possess immunosuppressive, antifungal and antiinflammatory activity in vivo and to inhibit thymocyte proliferation in vitro. Therefore, these formulations are useful in the treatment of Candida albicans infections, diseases of inflammation and transplant rejection autoimmune diseases, including lupus, rheumatoid arthritis, diabetes melitus, multiple sclerosis, etc.

Because the formulations disclosed herein contain rapamycin, they are considered to have antitumor, antifungal and antiproliferative activities. As such, the formulations of this invention are useful in the treatment of transplantation rejection, such as heart, kidney, liver, bone marrow and skin transplants; autoimmune diseases such as lupus, rheumatoid arthritis, diabetes mellitus, myasthenia gravis and multiple sclerosis; diseases of inflammation such as psoriasis, dermatitis, eczema, seborrhea, inflammatory bowel disease and eye uveitis; solid tumors; fungal infections; and hyperproliferative vascular diseases, such as restenosis. The present invention, therefore, also provides formulations useful for inducing immunosuppression in a mammal in such need. Such inducements would comprise administering to said mammal an immunosuppressive amount of one or more of the formulations discussed herein.

The present formulations comprise a rapamycin solution containing an organic solvent and phospholipid such as lecithin.

The formulations of the present invention may be produced as a one component, ready to use solution of rapamycin in a non-aqueous system consisting of a solvent and phospholipid to produce an acceptable dosage form for chronic use in association with immunosuppressant therapy, as well as antitumor, antifungal and antiproliferative activities. The one component system may be adjusted to eliminate the solvent in cases where the drug concentration can be solubilized in the remaining ingredients. The invention may also be produced alternatively as a two component system either comprised of a dry component fill of 100% rapamycin and diluent or a drug concentrate and diluent. Other filler materials, such as lactose or mannitol, may be used as a portion of the dry component of such systems.

Accordingly this invention provides a composition of matter containing from 0.01 gram to 10.0 grams of rapamycin per 100 ml of composition, and a solvent system, said solvent system comprising from 0.05 to 10% by volume of a surfactant, from 0 to 25% by volume of inert solvent and from 65 to 99.95% by volume of phospholipid solution in which phospholipid in said solution is from 40% to 75% by weight.

In a first preferred aspect this invention provides a composition of matter as defined above comprising 0.01 to 10 grams of rapamycin per 100 mls of composition and a solvent system comprising one or more of the following:
a) 0.01 to 20% by volume of an inert solvent; preferably 0.5 to 20%; most preferably 1 to 10%; and/or
b) 0.1 to 10% by volume of surfactant; preferably 0.5 to 8%; most preferably 1 to 5%; and/or
c) 65 to 99.8% by volume of a phospholipid solution; preferably 72 to 99.0% most preferably 85 to 98%

In a second preferred aspect this invention provides a composition of matter comprising from 0.01 to 1.0 gram of rapamycin per 100 mls and a solvent system comprising one or more of the following:
a) 0.05% to 10% volume of surfactant, preferably 0.1% to 5% by volume, most preferably 0.5 to 5% by volume, and/or
b) 90 to 99.95% by volume of a phospholipid solution, preferably 95 to 99.9% by volume, most preferably 95 to 98.5% of a phospholipid solution.

It is further preferred that the phospholipid solution used comprises 40% to 60% by weight of phospholipid, most preferably about 50% by weight. The preferred phospholipid is lecithin. A preferred solvent for the phospholipid solution is propylene glycol but other solvents may be used as described herein.

In general, the formulations of the first aspect of this invention concern combinations of a) rapamycin, b) surfactant, c) N,N-dimethylacetamide (DMA) or other inert solvent and d) phospholipid e.g. lecithin in the following ranges (per 100 ml formulation):
a) rapamycin at a concentration of from 0.01 to 10.0 grams per 100 ml;
b) surfactant at a concentration of from 0.1 to 10.0 ml per 100 ml;
c) DMA at a concentration of from 0.1 to 25 ml per 100 ml; and
d) from 65 to 99.8 ml per 100 ml of a lecithin or phospholipid solution containing from 40 to 60 percent lecithin or phospholipid in suitable solvent.

More preferred formulations of the first aspect of the present invention include those combinations having the following ranges of materials:
a) rapamycin at a concentration of from 0.05 to 5.0 grams per 100 ml;
b) surfactant at a concentration of from 0.5 to 8.0 ml per 100 ml;
c) DMA at a concentration of from 0.5 to 20 ml per 100 ml; and
d) from 72 to 99.0 ml per 100 ml of a lecithin or phospholipid solution containing from 40 to 60 percent lecithin or phospholipid in suitable solvent.

The most preferred formulations of the first aspect of this invention include those having the following ranges of concentrations:
a) rapamycin at a concentration of from 0.10 to 1.0 gram per 100 ml;
b) surfactant at a concentration of from 1.0 to 5.0 ml per 100 ml;
c) DMA at a concentration of from 1.0 to 10 ml per 100 ml; and
d) from 85 to 98 ml per 100 ml of a lecithin or phospholipid solution containing from 40 to 60 percent lecithin or phospholipid in suitable solvent.

In general, the formulations or compositions of the second aspect of the present invention include those comprising combinations of a) rapamycin, b) surfactant, and c) lecithin or phospholipid in the following concentrations (per 100 ml formulation);
a) rapamycin at a concentration of from 0.01 grams to 1.0 gram per 100 ml;
b) Surfactant at a concentration of from 0.05 ml to 10 ml per 100 ml; and
c) from 90 to 99.95 ml per 100 ml of a lecithin or a phosholipid solution containing from 40 to 60 percent of lecithin or phospholipid in a suitable solvent.

More preferred formulations of the second aspect of the present invention include those having the following concentration (per 100 ml formulation):
a) rapamycin at a concentration of from 0.03 grams to 0.8 grams per 100 ml;
b) Surfactant at a concentration of from 0.10 ml to 5 ml per 100 ml; and
c) from 95 to 99.9 ml per 100 ml of a lecithin or a phosholipid solution containing from 40 to 60 percent of lecithin or phospholipid in a suitable solvent.

A most preferred formulation of the second aspect of the present invention includes those formulations having concentrations of ingredients within the following ranges:
a) rapamycin at a concentration of from 0.05 grams to 0.5 grams per 100 ml;
b) Surfactant at a concentration of from 0.5 ml to 5 ml per 100 ml; and
c) from 95 to 99.5 ml per 100 ml of a lecithin or a phosholipid solution containing from 40 to 60 percent of lecithin or phospholipid in a suitable solvent.

The examples provided below list a number of solvents that are useful as inert solvents or as solvent in the phospholipid solution for the formulations of the present invention. Alternate solvents that can be used include, but are not limited to, dimethylacetamide, ethanol, dimethylformamide, glycerin, t-butanol, polythylene glycol and propylene glycol. The amounts of the solvents can be raised in conjunction with the drug concentration(s). As another alternative, the amounts of the solvents can be reduced in conjunction with the drug concentration and, if drug solubility permits, the lecithin solution alone can act as the solvent.

Examples of surfactants in the compositions of matter according to the present invention are one or more of the following:
polyoxyethylene sorbitol esters, polyoxyethylated fatty acids, polyoxyethylated fatty alcohols and polyoxyethylated glycerin hydroxy fatty esters.

Particular surfactants that may be used with the present formulations include, but are not limited to, Polysorbate 20 (polyoxyethylene 20 sorbitan monolaurate), Polysorbate 60, Span 80® Sorbitan Oleate, a product of ICI Americas, Wilmington, DE, the Cremophor® surfactants produced by the BASF Corporation, Parsippany, NJ, and Polysorbate 80, which is defined by the Merck Index, 11th Edition, published by Merck & Co., Inc., Copyright 1989, on page 1254 as Sorbitan mono-9-octadecenoate poly(oxy- 1,2-ethanediyl) derivatives, polyoxyethylene (20) sorbitan mono-oleate, Sorbitan mono-oleate polyoxyethylene, Sorlate, Tween 80, among others, and indicates an oleate ester of sorbitol and its anhydrides copolymerized with approximately 20 moles of ethylene oxide for each mole of sorbitol and sorbitol anhydrides. Polysorbate 80 is the surfactant preferred with the present invention.

A number of lecithin or phospholipid solutions may be used in the present formulations. Lecithin is a general term for phosphatidylcholine or a mixture of various diglycerides of stearic, palmitic, and oleic acids, linked to the choline ester of phosphoric acid. Various types of lecithin or lecithin sourced products (such as separated phospholipids), either alone or mixed with various solvents wherein the lecithin comprises about 40-75%, e.g. about 40-60%, can be used as the final ingredient of the formulations mentioned above. These lecithin ingredients can include, for example, Alcolec® lecithin, produced by the American Lecithin Company, Danbury, CT, Phosal 50 PG propylene glycol and lecithin, Phosal 50 MCT phosphatidylcholine and medium chained triglycerides, and Phospholipan 90® lecithin, all of which are produced by Nattermann Phospholipid GMBH, Colone, Germany, the Centrophil® and Centrophase® lecithins produced by Central Soya, Fort Wayne, IN. It is preferred that the phospholipid solutions used in the present formulation have at least a 50% concentration of phospholipid. More particularly, it is preferred that the lecithin products or solutions used with the present formulations have at least 50% phosphatidylcholine. It is also preferred that the phospholipid solution comprise a phospholipid in propylene glycol.

The dosage requirements may vary the severity of the symptoms presented and the particular subject being treated. Projected daily oral dosages of the compounds of this invention would be 0.005 - 75 mg/kg, preferably between 0.01 - 50 mg/kg, and more preferably between 0.05 - 10 mg/kg.

Treatment will generally be initiated with small dosages less than the optimum dose of the compound. Thereafter the dosage is increased until the optimum effect under the circumstances is reached. Precise dosages will be determined by the administering physician based on experience with the individual subject treated. In general, the formulations of this invention are most desirably administered at a concentration that will generally afford effective results without causing any harmful or deleterious side effects.

The present formulations may be administered to the patient by the means generally used for oral liquid medications. They may be taken, by themselves, or they may be dispersed in a liquid, such as water or juices. The formulations may also be capsulized, such as in starch capsules or soft elastic gelatin capsules. Rapamycin oral may be dispersed into water for dosing in the range of about 1 part of formula into about 9 parts water downward to about 1 part of formula into about 499 parts water by mixing for a minimum of about 60 seconds. This dispersion may be used over about a 1 hour period with mixing prior to dosing.

It is contemplated that when the formulations of this invention are used as an immunosuppressive or antiinflammatory agent, they can be administered in conjunction with one or more other immunoregulatory agents. Such other antirejection chemotherapeutic agents include, but are not limited to azathioprine, corticosteroids, such as prednisone and methylprednisolone, cyclophosphamide, cyclosporin A, FK-506, OKT-3, and ATG. By combining one or more of the formulations of the present invention with such other drugs or agents for inducing immunosuppression or treating inflammatory conditions, lesser amounts of each of the agents may be required to achieve the desired effect. The basis for such combination therapy was established by Stepkowski whose results showed that the use of a combination of rapamycin and cyclosporin A at subtherapeutic doses significantly prolonged heart allograft survival time. [Transplantation Proc. 23:507 (1991)].

This invention also provides a process for preparing a composition of matter which comprises dissolving rapamycin in one or more components of a solvent system comprising from 0.05 to 10% by volume of surfactant, from 0 to 25% by volume of inert solvent and from 65 to 99.95% by volume of phospholipid solution in which phospholipid in said solution is from 40% to 75% by weight such that the concentration of rapamycin in the total composition is from 0.01 to 10.0 grams per 100ml.

It is also understood that the present formulations may be used with other ingredients used with conventional oral formulations such as, but not limited to, flavor enhancers, coloring agents, adjuvants, antifungal agents, antibacterial agents, etc.

The following non-limiting examples and comparative examples are provided to illustrate the effectiveness of the more preferred embodiments of the present invention.

### EXAMPLES

### EXAMPLE 1

The following Example 1 demonstrates an oral rapamycin formulation having a concentration of rapamycin which is 50mg/kg.

| A. Formula: | |
|---|---|
| Ingredients | Amount |
| Rapamycin @ 100% up to | 5.0 gm |
| Polysorbate 80, NF | 5.0 ml or 5.4 gm |
| N,N-dimethylacetamide | 20.0 ml or 18.7 gm |
| Phosal® 50 PG¹ q.s. | 100 ml or 99.6 gm |

| | |
|---|---|
| ¹Nattermann brand of lecithin and propylene glycol | |

### Manufacturing Directions:

1. Weigh the rapamycin into a suitable container.
2. Add the N,N-dimethylacetamide to the container in Step #1. Mix until dissolved.
3. Add the Polysorbate 80 to the container in Step #2. Mix until uniform.
4. Adjust to the final volume with Phosal 50 PG® lecithin and propylene glycol.
5. Mix until uniform

Two Cynomolgus monkeys, listed below as A and B, were administered the above formulation at a dose of 50 mg/kg of rapamycin and the following serum concentrations were determined at the indicated time after dosing.

| Rapamycin Concentration in Monkey Serum Dosed Orally with 50 mg/kg | | |
|---|---|---|
| Time | A | B |
| 0 | BDL | BDL |
| 1 h | 0.017 | 0.035 |
| 2 h | 0.037 | 0.166 |
| 3 h | 0.062 | 0.078 |
| 4 h | 0.215 | 0.115 |
| 6 h | 0.262 | 0.050 |
| 9 h | 0.103 | 0.010 |
| 12 h | 0.018 | BDL |
| BDL = Below detection limit (detection limit ∼ or equal to 0.006 µg/ml) | | |

### EXAMPLE 2

The following Example 2 provides a oral formulation having a rapamycin concentration of 125 mg/ml, as well as the procedure for its preparation. The first set of ingredients and procedures provided demonstrate the production of an oral rapamycin concentrate. The second set of ingredients and procedures provided demonstrate a diluent which may be used with the rapamycin concentrate.

### Rapamycin Oral Concentration at 125 mg/ml in DMA

| Formula: | |
|---|---|
| Ingredients | Amount |
| Rapamycin @ 100% | 12.5 gm |
| Dimethylacetamide (DMA) q.s. | 100 ml |

### Procedure:

1. Weigh 12.5 g of rapamycin into a suitably calibrated container.
2. Q.S. to 100 ml with DMA.
3. Mix until a clear solution is formed.
4. Store rapamycin concentrate in all glass container or in a flint glass vial stoppered with a Teflon barrier faced stopper.

The following Diluent No. 1 is used in the oral rapamycin formula (rapamycin at 25 mg/ml) which follows:

### Diluent No. 1 for Oral Rapamycin

| Formula: | |
|---|---|
| Ingredients | Amount |
| Polysorbate 80 | 6.69 gm |
| Centrophil® W¹ q.s. | 100 ml |

| | |
|---|---|
| ¹Central Soya brand of lecithin | |

### Procedure:

1. Add 6.69 grams of Polysorbate 80 to a suitable container
2. Q.S. to 100 ml with Centrophil W® lecithin.
3. Mix until homogeneous.
4. Diluent for oral rapamycin can be stored in an all glass container or in a flint glass vial stoppered with a Teflon barrier faced stopper at room temperature.

### Rapamycin Oral at 25 mg/ml

| Formula: | |
|---|---|
| Ingredients | Amount |
| Rapamycin Oral Concentrate @ 125 mg/ml | 20 ml |
| Diluent for Oral Rapamycin q.s. | 100ml |

### Procedure:

1. Place 20 ml of rapamycin oral concentrate into a container.
2. Q.S. to 100 ml with diluent for oral rapamycin.
3. Mix until homogeneous.
4. This rapamycin formula can be stored in an all glass container or in a flint glass vial stoppered with a Teflon barrier faced stopper.

Four Cynomolgus monkeys, listed below as A-D, were administered the above formulation at a dose of 50 mg/kg of rapamycin and the following serum concentrations were determined at the indicated time after dosing.

### Rapamycin Concentration in Monkey Serum Dosed Orally with 50 mg/kg

| Rapamycin Concentration (µg/ml) Monkey No. | | | | |
|---|---|---|---|---|
| Time | A | B | C | D |
| 0 | BDL | BDL | BDL | BDL |
| 1 h | 0.008 | 0.786 | 0.078 | 0.053 |
| 2 h | 0.020 | 0.129 | 0.066 | 0.013 |
| 3 h | 0.026 | 0.077 | 0.101 | 0.022 |
| 4 h | 0.104 | 0.036 | >0.200 | 0.057 |
| 6 h | QNS | 0.029 | >0.200 | 0.117 |
| 9 h | 0.113 | 0.012 | >0.200 | 0.031 |
| 12 h | 0.022 | 0.005 | 0.050 | 0.005 |
| QNS = Quantity not sufficient. BDL = Below detection limit (detection limit ∼ or equal to µg/ml) | | | | |

The procedural steps for formulation and storage of the 5 mg/ml oral rapamycin formulation are the same as those listed in Example 1, as are the alternative order of addition of ingredients and the methods of comminution.

### EXAMPLE 3

### Rapamycin Oral at 1 mg/ml

A rapamycin oral formulation at a concentration of 1 mg/ml can be formulated from the following active and inactive ingredients by the procedural steps which follow:

| Active Ingredient: | Conc. | Input | Batch Formula 10.000 bottles |
|---|---|---|---|
| Rapamycin @ 100% | 1.00 mg/ml | 0.025 g | 0.250 kg |

| Inactive Ingredients: | | | |
|---|---|---|---|
| Polysorbate 80, NF | 10.8 mg/ml | 0.270 g | 2.700 kg |
| Phosal 50 PG® propylene glycol and lecithin q.s. ad | 1.00 ml | 25.0 ml | 250.0 l |
| or | 1.005 gm or | 25.125 g | 251.25 kg |

Density of the Final Formulation - 1.005 g/ml

If the potency of the rapamycin is less than 100%, the input must be adjusted to achieve the claimed potency.

### Method of Manufacture

### Procedure:

1. Weigh the rapamycin into a suitable container.
2. Add the Polysorbate 80 to the container in step #1
3. Adjust to the final volume with Phosal® 50 PG.
4. Mix until the rapamycin is dissolved.
5. Fill 25 ml ± 1.25 ml (25.125 g ± 1.256 g) into each one ounce amber glass bottle. It is preferable to seal with a child resistant cap.

For improved wettability and ease of solution, an alternative order of addition of the ingredients and amounts presented above is as follows:
1. Polysorbate 80.
2. A portion of the Phosal® 50 PG propylene glycol and lecithin.
3. Rapamycin.
4. The remaining Phosal® 50 PG propylene glycol and lecithin.

The rapamycin in these formulations may also be comminuted by use of a mill or mortar and pestle and passed through an 80 mesh screen.

### EXAMPLE 4

### Rapamycin Oral at 5 mg/ml

A rapamycin oral formulation at a concentration of 5 mg/ml can be formulated from the following active and inactive ingredients by the procedural steps which follow:

| Active Ingredient: | Conc. | Input | Batch Formula 10.000 bottles |
|---|---|---|---|
| Rapamycin @ 100% | 5.00 mg | 0.125 g | 1.250 kg |

| Inactive Ingredients: | | | |
|---|---|---|---|
| Polysorbate 80, NF | 10.8 mg | 0.270 g | 2.70 kg |
| Phosal® 50 PG propylene glycol and lecithin q.s. ad | 1.00 ml | 25.0 ml | 250.0 l |
| or | 1.005 gm or | 25.125 g or | 251.25 kg |

Density of the Final Formulation - 1.005 g/ml.

If the potency of the rapamycin is less than 100%, the input must be adjusted to give the claimed potency.

The procedural steps for formulation and storage of the 5 mg/ml oral rapamycin formulation are the same as those listed in Example 3, as are the alternative order of addition of ingredients and the methods of comminution.

### EXAMPLE 5

The formulation of this Example was produced using the ingredients which follow and the methods indicated below:

| Ingredients | Amount |
|---|---|
| Rapamycin @ 100% up to | 1.0 gm |
| Polysorbate 80, NF | 1.0 ml or 1.08 gm |
| Phosal 50® PG lecithin and propylene glycol q.s. | 100 ml or 100.5 mg |

### Method of Formulation

1. Weigh the rapamycin into a suitable container.
2. Add the Polysorbate 80 into the container of Step #1.
3. Adjust to the final volume with Phosal 50 PG® propylene glycol and lecithin.
4. Mix until a solution results.

Alternatively, this formula can be packaged in a suitable container or encapsulated into a capsule.

### EXAMPLE 6

The oral formulations of this invention, such as those disclosed in the Examples above, may also be prepared in encapsulated forms, such as formulations within starch capsules. The following procedure describes a method which may be utilized to prepare such encapsulated formulations.

### Procedure:

1) Add to a container, NF, the Polysorbate 80.
2) Add to the Polysorbate 80 of Step #1 80% of the the required Phosal® 50 PG.
3) Weigh the rapamycin component of the formulation into the container of Step #2.
4) Adjust to the final formulation weight with Phosal® 50 PG.
5) Establish a nitrogen atmosphere over the formulation and maintain until the capsules are filled.
6) Mix the formulation until the rapamycin is dissolved.
7) Pass the formulation solution through a particulate (such as a 100 mesh screen) or scintered glass filter.
8) Fill 0.50 ml of the Step #7 material into capsule shells using an automatic syringe dispensing unit and seal the capsule.
9) Package the filled capsules upon completion of encapsulation. An example of a preferred package is a conventional blister package with a perforable metal foil backing.
10) Store the finished encapsulated product at refridgerated conditions (2-8°C) protected from light.

The primary capsule sealant for the starch capsule may be a 5% Dextrin, NF, aqueous solution. It is preferable to heat purified water to 50-60° C prior to compounding to facilitate dissolution of the Dextrin. Prior to use it is also preferable to filter the the Dextrin solution through a suitable particulate filter.

### Bioavailability

a) Cynomolgus monkeys were administered a starch encapsulated formulation of Example 5, above, at a dose of 0.25 mg/kg of rapamycin and the following serum concentrations were determined at the indicated time after dosing:

### Rapamycin Concentration in Monkey Serum Dosed Orally as a Dispersion of 0.25mg/kg

| Rapamycin Concentrate (µg/ml) | | | | | | |
|---|---|---|---|---|---|---|
| Time (h) | A | B | C | D | E | F |
| 0 | 0.000 | 0.000 | 0.000 | 0.000 | 0.000 | |
| | 0.000 | | | | | |
| .25 | 0.012 | 0.001 | 0.005 | 0.000 | 0.000 | |
| | 0.000 | | | | | |
| .50 | 0.014 | 0.000 | 0.024 | 0.004 | 0.000 | |
| | 0.003 | | | | | |
| 1 | 0.011 | 0.002 | 0.021 | 0.006 | 0.003 | |
| | 0.004 | | | | | |
| 2 | 0.005 | 0.019 | 0.008 | 0.004 | 0.007 | |
| | 0.003 | | | | | |
| 4 | 0.002 | 0.006 | 0.007 | 0.003 | 0.006 | |
| | 0.002 | | | | | |
| 8 | 0.002 | 0.004 | 0.005 | 0.003 | 0.002 | |
| | 0.001 | | | | | |
| 12 | 0.001 | 0.002 | 0.003 | 0.002 | 0.001 | |
| | 0.001 | | | | | |
| 24 | 0.001 | 0.002 | 0.001 | 0.001 | 0.002 | |
| | 0.001 | | | | | |
| 36 | 0.000 | 0.002 | 0.001 | 0.001 | 0.000 | |
| | 0.000 | | | | | |

### Rapamycin Concentration in Monkey Serum Dosed Orally in Starch Capsules 0.25mg/kg

| Rapamycin Concentrate (µg/kg) | | | | | | |
|---|---|---|---|---|---|---|
| Time (h) | A | B | C | D | E | F |
| 0 | 0.000 | 0.000 | 0.000 | 0.000 | 0.000 | |
| | 0.000 | | | | | |
| .25 | 0.000 | 0.000 | 0.000 | 0.000 | 0.000 | |
| | 0.000 | | | | | |
| .50 | 0.000 | 0.000 | 0.005 | 0.000 | 0.005 | |
| | 0.000 | | | | | |
| 1 | 0.029 | 0.004 | 0.026 | ------- | 0.008 | |
| | 0.000 | | | | | |
| 2 | 0.011 | 0.019 | 0.032 | 0.000 | 0.011 | |
| | 0.004 | | | | | |
| 4 | 0.007 | 0.009 | 0.011 | 0.002 | 0.007 | |
| | 0.002 | | | | | |
| 8 | 0.004 | 0.003 | 0.004 | 0.002 | 0.005 | |
| | 0.002 | | | | | |
| 12 | 0.002 | 0.001 | ------- | 0.001 | 0.002 | |
| | 0.001 | | | | | |
| 24 | 0.001 | 0.000 | 0.002 | 0.001 | 0.001 | |
| | 0.000 | | | | | |
| 36 | 0.000 | 0.000 | 0.000 | 0.000 | 0.000 | |
| | 0.000 | | | | | |

### Rapamycin Concentration in Monkey Serum Dosed Orally in SEG Capsules at 0.25 mg/kg

| Time (h) | A | B | C | D | E | F |
|---|---|---|---|---|---|---|
| 0 | 0.000 | 0.000 | 0.000 | 0.000 | 0.000 | |
| | 0.000 | | | | | |
| .25 | 0.005 | 0.002 | 0.001 | 0.001 | 0.001 | |
| | 0.002 | | | | | |
| .50 | 0.001 | 0.001 | 0.002 | 0.002 | 0.001 | |
| | 0.001 | | | | | |
| 1 | 0.043 | 0.022 | 0.019 | 0.002 | 0.003 | |
| | 0.012 | | | | | |
| 2 | 0.027 | 0.030 | 0.019 | 0.002 | 0.010 | |
| | 0.008 | | | | | |
| 4 | 0.012 | 0.012 | 0.015 | 0.009 | 0.011 | |
| | 0.006 | | | | | |
| 8 | 0.008 | 0.006 | 0.009 | 0.004 | 0.006 | |
| | 0.003 | | | | | |
| 12 | 0.008 | 0.004 | 0.006 | 0.002 | 0.005 | |
| | 0.002 | | | | | |
| 24 | 0.006 | 0.003 | 0.005 | 0.001 | 0.002 | |
| | 0.001 | | | | | |
| 36 | 0.002 | 0.001 | 0.002 | 0.001 | 0.002 | |
| | 0.001 | | | | | |

b) 3 mg starch encapsulated formulations containing rapamycin at a concentration of 6 mg/ml, prepared as described above, were administered to 14 healthy male human volunteers between the ages of 18 and 45, from whom blood samples were drawn at the time intervals indicated in the table below. The rapamycin blood samples were assayed for whole blood rapamycin concentration using a validated (ESP)-HPLC-MS method.

| Time Interval Following Administration (Hours) | Blood Concentration (conc. = ng/ml) |
|---|---|
| 0.33 | 0.41 |
| 0.67 | 6.53 |
| 1 | 8.57 |
| 2 | 8.27 |
| 3 | 5.54 |
| 4 | 3.96 |
| 5 | 3.10 |
| 8 | 1.93 |
| 12 | 1.47 |
| 18 | 1.05 |
| 24 | 0.80 |
| 48 | 0.54 |

### EXAMPLE 7

| Formula | Ingredients |
|---|---|
| Rapamycin @ 100% up to | 2.5 grams |
| Polysorbate 80, NF | 5.0 ml or 5.4 gm |
| Absolute Ethanol | 12.67 ml or 10.0 gm |
| Phosal 50® PG lecithin and propylene glycol q.s. | 100 ml |

This formulation can be produced by the following steps:
1. Weigh the rapamycin into a suitable container
2. Add the absolute ethanol to the container in Step #1. Mix until dissolved.
3. Add the polysorbate 80 to the container in Step #2. Mix until uniform.
4. Add Phosal 50® PG lecithin and propylene glycol to adjust to the final volume.
5. Mix until uniform.

Alternatively, this formula can be packaged in a suitable container or encapsulated into a capsule.

Cynomolgus monkeys were administered the formulation above at a dose of 0.25 mg/kg of rapamycin and the following serum concentrations were determined at the indicated time after dosing.

### Rapamycin Concentration in Monkey Serum Dosed Orally in a Dispersion at 0.25 mg/kg

| Rapamycin Concentration (µg/ml) Monkey No. | | | | | | |
|---|---|---|---|---|---|---|
| Time (h) | A | B | C | D | E | F |
| 0 | 0.000 | 0.000 | 0.000 | 0.000 | 0.000 | 0.000 |
| .25 | --- | 0.025 | 0.007 | 0.010 | 0.007 | 0.003 |
| .50 | 0.008 | 0.030 | 0.027 | 0.004 | 0.016 | 0.012 |
| 1 | 0.050 | 0.022 | 0.051 | 0.006 | 0.051 | 0.011 |
| 2 | 0.026 | 0.026 | 0.026 | 0.019 | 0.025 | 0.006 |
| 4 | 0.008 | 0.011 | 0.020 | 0.005 | 0.018 | 0.006 |
| 8 | 0.008 | 0.004 | 0.009 | 0.003 | 0.011 | 0.003 |
| 12 | 0.004 | 0.002 | 0.006 | 0.005 | 0.007 | 0.003 |
| 24 | 0.002 | --- | 0.004 | 0.002 | 0.004 | 0.001 |
| 36 | 0.000 | 0.003 | 0.003 | 0.001 | 0.003 | 0.002 |

### Rapamycin Concentration in Monkey Serum Dosed Orally in SEG Capsules at 0.25 mg/kg

| Rapamycin Concentration (µg/ml) Monkey No. | | | | | | |
|---|---|---|---|---|---|---|
| Time (h) | A | B | C | D | E | F |
| 0 | 0.000 | 0.000 | 0.000 | 0.000 | 0.000 | 0.000 |
| .25 | 0.000 | 0.000 | 0.000 | 0.000 | 0.001 | 0.001 |
| .50 | 0.000 | 0.000 | 0.000 | 0.000 | 0.010 | 0.006 |
| 1 | 0.030 | 0.013 | 0.001 | 0.000 | 0.019 | 0.005 |
| 2 | 0.014 | 0.024 | 0.014 | 0.002 | 0.014 | 0.005 |
| 4 | 0.013 | 0.011 | 0.003 | 0.006 | 0.015 | 0.004 |
| 8 | 0.007 | 0.004 | 0.002 | 0.002 | 0.007 | 0.002 |
| 12 | 0.005 | 0.003 | 0.001 | 0.001 | 0.006 | 0.001 |
| 24 | 0.003 | 0.001 | 0.001 | 0.001 | 0.003 | 0.001 |
| 36 | 0.002 | 0.001 | 0.001 | 0.000 | 0.001 | 0.000 |

### COMPARATIVE EXAMPLES

### COMPARATIVE EXAMPLE 1

The following traditional formulation approaches applied to rapamycin are provided as a comparison to those of the present invention. The ingredients and manufacturing directions for Diluent No. 2, below, are used to create the diluent in the comparative oral formulation (Rapamycin Oral Suspension at 50 mg/ml) which follows:

### Diluent for Rapamycin Suspension

| Ingredients | Amount |
|---|---|
| Polysorbate 80, NF | 5.0 ml |
| 0.5 M Citric Acid (pH 4) q.s. | 100 ml |

### Manufacturing Directions:

1. Prepare a 0.5 M citric acid solution.
2. Adjust the pH of the solution in Step #1 to 4.0 using 50% w/w NaOH.
3. Place the Polysorbate 80 into a suitable container.
4. QS to 100 ml with solution from step #2..
5. Mix until uniform.

### Rapamycin Oral Suspension at 50 mg/ml

| Ingredients | Amount |
|---|---|
| Rapamycin Micronized @ 100% up to | 5.0 gm |
| Diluent for Rapamycin Oral Suspension q.s. | 100 ml |

### Manufacturing Directions:

1. Weigh the rapamycin into a suitable container.
2. QS with the diluent for rapamycin
3. Mix until uniform.

Three Cynomolgus monkeys, listed below as A-C, were administered the above formulation at a dose of 50 mg/kg of rapamycin and the following serum concentrations were determined at the indicated time after dosing.

### Rapamycin Concentration in Monkey Serum Dosed Orally with 50 mg/kg Rapamycin Oral Suspension

| Rapamycin Concentration (µg/ml) Monkey No. | | | |
|---|---|---|---|
| Time | A | B | C |
| 0 | BDL | BDL | BDL |
| 1 h | BDL | BDL | BDL |
| 2 h | BDL | BDL | BDL |
| 3 h | BDL | BDL | BDL |
| 4 h | BDL | BDL | BDL |
| 6 h | BDL | BDL | BDL |
| 9 h | BDL | BDL | BDL |
| 12 h | BDL | BDL | BDL |
| BDL = Below detection limit (detection limit ∼ or equal to 0.006 µg/ml). | | | |

### COMPARATIVE EXAMPLE 2

The following ingredients and procedural steps demonstrate the production of another traditional approach which has been applied to form an oral rapamycin solution, which is provided for comparison with the present invention.

### Rapamycin Oral Solution at 50 mg/ml

| Formula: | |
|---|---|
| Ingredients | Amount |
| Rapamycin @ 100% | 5.0 gm |
| Dimethylacetamide | 10.0 gm |
| Absolute Ethanol | 10.0 gm |
| Miglyol 812 q.s. | 100 ml |

### Procedure:

1. Place rapamycin into a suitable container.
2. Add the dimethylacetamide and ethanol to the container in Step #1 and mix until a solution results.
3. QS with Miglyol 812 and mix until uniform.
4. (Alternative Step) Filter sample through a 0.2 micron Teflon filter.

Three Cynomolgus monkeys, listed below as A-C, were administered the above formulation at a dose of 50 mg/kg of rapamycin and the following serum concentrations were determined at the indicated time after dosing.

### Rapamycin Concentration in Monkey Serum Dosed Orally with 50 mg/kg Rapamycin Oral Solution

| Rapamycin Concentration (µg/ml) Monkey No. | | | |
|---|---|---|---|
| Time | A | B | C |
| 0 | BDL | BDL | BDL |
| 1 h | BDL | BDL | BDL |
| 2 h | BDL | BDL | BDL |
| 3 h | BDL | BDL | BDL |
| 4 h | BDL | BDL | BDL |
| 6 h | BDL | BDL | BDL |
| 9 h | BDL | BDL | BDL |
| 12 h | BDL | BDL | BDL |
| BDL = Below detection limit (detection limit ∼ or equal to 0.006 µg/ml). | | | |

### COMPARATIVE EXAMPLE 3

### Rapamycin Oral Emulsion at 50 mg/ml

| Formula: | |
|---|---|
| Ingredients | Amount |
| Rapamycin @ 100% | 5.0 gm |
| Dimethylacetamide | 10 ml |
| Olive Oil q.s. | 100 ml |

### Procedure:

1. Place the rapamycin into a suitable container.
2. Add the dimethylacetamide to the container in Step #1 and mix until clear.
3. QS with olive oil and mix until homogenous.

Three Cynomolgus monkeys, listed below as A-C, were administered the above formulation at a dose of 50 mg/kg of rapamycin and the following serum concentrations were determined at the indicated time after dosing.

### Rapamycin Concentration in Monkey Serum Dosed Orally with 50 mg/kg Rapamycin Oral Emulsion

| Rapamycin Concentration (µg/ml) | | | |
|---|---|---|---|
| Time | A | B | C |
| 0 | BDL | BDL | BDL |
| 20 min | BDL | BDL | BDL |
| 40 min | BDL | BDL | BDL |
| 80 min | BDL | BDL | BDL |
| 3 h | BDL | BDL | BDL |
| 6 h | BDL | 0.110* | BDL |
| 12 h | BDL | BDL | BDL |
| 24 h | BDL | BDL | BDL |
| BDL = Below detection limit (detection limit ∼ or equal to 0.006 µg/ml). | | | |

| | | | |
|---|---|---|---|
| *assay result obtained from test lab appears aberent | | | |

## Claims

1. A composition of matter containing from 0.01 gram to 10.0 grams of rapamycin per 100 ml of composition, and a solvent system, said solvent system comprising from 0.05 to 10% by volume of surfactant, from 0 to 25% by volume of inert solvent and from 65 to 99.95% by volume of phospholipid solution in which the phospholipid in said solution is from 40% to 75% by weight.

2. A composition of matter according to Claim 1 containing from 0.01 to 10.0 grams of rapamycin per 100 ml of composition and a solvent system, said solvent system comprising 0.1 to 10% by volume of surfactant, from 0.1 to 25% by volume of inert solvent, and from 65 to 99.8% by volume of a phospholipid solution in which the phospholipid in said solution is from about 40 to about 60% by weight.

3. A composition of matter according to Claim 2 containing 0.05 to 5.0 grams of rapamycin per 100 ml of composition, and a solvent system comprising from 0.5 to 8.0% by volume of surfactant, from 0.5 to 20% by volume of inert solvent and from 72 to 99.0% by volume of phospholipid solution.

4. A composition of matter according to Claim 2 containing 0.1 to 1.0 grams of rapamycin per 100 ml of composition, and a solvent system comprising from 1.0 to 5.0% by volume of surfactant, from 1.0 to 10% by volume of an inert solvent and from 85 to 98.0% by volume of phospholipid solution.

5. A composition of matter according to Claim 1 containing from 0.01 to 1.0 gram of rapamycin per 100 ml of composition and a solvent system said solvent system comprising from 0.05% to 10% by volume of surfactant, and from 90% to 99.95% by volume of a phospholipid solution wherein the phospholipid in said solution is from 40% to 60% by weight.

6. A composition of matter according to Claim 5 containing from 0.03 to 0.8 gram of rapamycin per 100 ml of composition and a solvent system said solvent system comprising from 0.10% to 5% by volume of surfactant, and from 95% to 99.9% by volume of a phospholipid solution wherein the phospholipid in said solution is from 40% to 60% by weight.

7. A composition of matter according to Claim 6 containing from 0.05 to 0.5 gram of rapamycin per 100 ml of composition and a solvent system, said solvent system comprising from 0.5% to 5% by volume of surfactant, and from 95% to 98.5% by volume of a phospholipid solution wherein the phospholipid in said solution is from 40% to 60% by weight.

8. A composition of matter according to Claim 5 containing about 1.0 gram of rapamycin in a solvent system comprising about 1% by volume of surfactant and about 99% by volume of a phospholipid solution containing 50% by weight of phospholipid.

9. A composition of matter according to any one of Claims 1 to 7 wherein the phospholipid is about 50% by weight of the phopholipid solution.

10. A composition of matter according to any one of Claims 1 to 9 wherein the phospholipid is lecithin.

11. A composition of matter according to any one of Claims 1 to 10 wherein the surfactant is selected from one of the following: polyoxyethylene sorbitol esters, polyoxyethylated fatty acids, polyoxyethylated fatty alcohols and polyoxyethylated glycerin hydroxy fatty esters.

12. A composition of matter according to any one of Claims 1 to 11 wherein the inert solvent if present is selected from one or more of dimethylacetamide, ethanol, glycerin, dimethylformamide, t-butanol, polyethylene glycol and propylene glycol.

13. A composition of matter according to any one of Claims 1 to 12 wherein the solvent for the phospholipid solution is propylene glycol.

14. A composition of matter comprising, per 100 ml of the composition,
a) a first 20 ml component of 2500 mg of rapamycin in N,N-dimethylacetamide; and
b) a second component of from 0.05 gm/ml to 0.07 gm/ml of surfactant in lecithin, the second component being added to the first 20 ml component to complete a 100 ml composition volume.

15. A composition of matter comprising, per 100 ml composition, 2.5 grams of rapamycin, 5.0 ml of surfactant, about 13 ml of absolute ethanol, and a 50% lecithin or phospholipid solution q.s to 100 ml.

16. A composition of matter according to any one of Claims 1-15 when in unit dosage form.

17. A composition of matter according to Claim 16 in which the unit dosage form is a liquid filled capsule.

18. A process for preparing a composition of matter which comprises dissolving rapamycin in one or more components of a solvent system comprising from 0.05 to 10% by volume of surfactant, from 0 to 25% by volume of inert solvent and from 65 to 99.95% by volume of phospholipid solution in which phospholipid in said solution is from 40% to 75% by weight such that the concentration of rapamycin in the total composition is from 0.01 to 10.0 grams per 100ml.

19. A process for preparing a composition of matter according to Claim 16 which comprises dissolving rapamycin in one or more components of a solvent system comprising 0.1 to 10% by volume of surfactant, from 0.1 to 25% by volume of inert solvent, and from 65 to 99.8% by volume of a phospholipid solution in which the phospholipid in said solution is from 40 to 60% by weight such that the concentration of rapamycin in the total composition is from 0.01 to 10.0 grams per 100ml.

20. A process for preparing a composition of matter according to Claim 16 which comprises dissolving rapamycin in one or more components of a solvent system comprising from 0.05% to 10% by volume of surfactant, and from 90% to 99.95% by volume of a phospholipid solution wherein the phospholipid in said solution is from 40% to 60% by weight such that the concentration of rapamycin in the total composition is from 0.01 to 1.0 grams per 100ml.

## Patentansprüche

1. Substanzzusammensetzung_{,} welche 0,01 g bis 10,0 g Rapamycin pro 100 ml Zusammensetzung und ein Lösungsmittelsystem enthält, wobei das Lösungsmittelsystem 0,05 bis 10 Vol.% Surfactant, 0 bis 25 Vol.% inertes Lösungsmittel und 65 bis 99,95 Vol.% Phospholipid-Lösung umfaßt, worin das Phospholipid in der Lösung 40 bis 75 Masse-% ausmacht.

2. Substanzzusammensetzung nach Anspruch 1, welche 0,01 g bis 10,0 g Rapamycin pro 100 ml Zusammensetzung und ein Lösungsmittelsystem enthält, wobei das Lösungsmittelsystem 0,1 bis 10 Vol.% Surfactant, 0,1 bis 25 Vol.% inertes Lösungsmittel und 65 bis 99,8 Vol.% einer Phospholipid-Lösung umfaßt, worin das Phospholipid in der Lösung 40 bis etwa 60 Masse-% ausmacht.

3. Substanzzusammensetzung nach Anspruch 2, welche 0,05 bis 5,0 g Rapamycin pro 100 ml Zusammensetzung und ein Lösungsmittelsystem enthält, das 0,5 bis 8,0 Vol.% Surfactant, 0,5 bis 20 Vol.% inertes Lösungsmittel und 72 bis 99,0 Vol.% Phospholipid-Lösung umfaßt.

4. Substanzzusammensetzung nach Anspruch 2, welche 0,1 bis 1,0 g Rapamycin pro 100 ml Zusammensetzung und ein Lösungsmittelsystem enthält, das 1,0 bis 5,0 Vol.% Surfactant, 1,0 bis 10 Vol.% eines inerten Lösungsmittels und 85 bis 98,0 Vol.% Phospholipid-Lösung umfaßt.

5. Substanzzusammensetzung nach Anspruch 1, welche 0,01 bis 1,0 g Rapamycin pro 100 ml Zusammensetzung und ein Lösungsmittelsystem enthält, wobei das Lösungsmittelsystem 0,05 Vol.% bis 10 Vol.% Surfactant und 90 Vol.% bis 99,95 Vol.% einer Phospholipid-Lösung umfaßt, worin das Phospholipid in der Lösung 40 Masse-% bis 60 Masse-% ausmacht.

6. Substanzzusammensetzung nach Anspruch 5, welche 0,03 bis 0,8 g Rapamycin pro 100 ml Zusammensetzung und ein Lösungsmittelsystem enthält, wobei das Lösungsmittelsystem 0,10 Vol.% bis 5 Vol.% Surfactant und 95 Vol.% bis 99,9 Vol.% einer Phospholipid-Lösung umfaßt, worin das Phospholipid in der Lösung 40 Masse-% bis 60 Masse-% ausmacht.

7. Substanzzusammensetzung nach Anspruch 6, welche 0,05 bis 0,5 g Rapamycin pro 100 ml Zusammensetzung und ein Lösungsmittelsystem enthält, wobei das Lösungsmittelsystem 0,5 Vol.% bis 5 Vol.% Surfactant und 95 Vol.% bis 98,5 Vol.% einer Phospholipid-Lösung umfaßt, worin das Phospholipid in der Lösung 40 Masse-% bis 60 Masse-% ausmacht.

8. Substanzzusammensetzung nach Anspruch 5, welche etwa 1,0 g Rapamycin in einem Lösungsmittelsystem umfaßt, das etwa 1 Vol.% Surfactant und etwa 99 Vol.% einer Phospholipid-Lösung umfaßt, die 50 Masse-% Phospholipid enthält.

9. Substanzzusammensetzung nach einem der Ansprüche 1 bis 7, worin das Phospholipid etwa 50 Masse-% der Phospholipid-Lösung ausmacht.

10. Substanzzusammensetzung nach einem der Ansprüche 1 bis 9, worin das Phospholipid Lecithin ist.

11. Substanzzusammensetzung nach einem der Ansprüche 1 bis 10, worin das Surfactant aus einem der folgenden ausgewählt ist: Polyoxyethylensorbitestern, polyoxyethylierten Fettsäuren, polyoxyethylierten Fettalkoholen und polyoxyethylierten Glycerinhydroxyfettsäureestern.

12. Substanzzusammensetzung nach einem der Ansprüche 1 bis 11, worin das inerte Lösungsmittel, wenn vorhanden, ausgewählt ist aus einem oder mehreren von Dimethylacetamid, Ethanol, Glycerin, Dimethylformamid, tert.Butanol, Polyethylenglykol und Propylenglykol.

13. Substanzzusammensetzung nach einem der Ansprüche 1 bis 12, worin das Lösungsmittel für die Phospholipid-Lösung Propylenglykol ist.

14. Substanzzusammensetzung, welche pro 100 ml der Zusammensetzung umfaßt:
a) eine erste 20 ml Komponente von 2500 mg Rapamycin in N,N-Dimethylacetamid; und
b) eine zweite Komponente von 0,05 g/ml bis 0,07 g/ml Surfactant in Lecithin, wobei die zweite Komponente der ersten 20 ml Komponente zugesetzt wird, um eine 100 ml Zusammensetzungsvolumen zu vervollständigen.

15. Substanzzusammensetzung, welche pro 100 ml Zusammensetzung 2,5 g Rapamycin, 5,0 ml Surfactant, etwa 13 ml absolutes Ethanol und eine 50 % Lecithin- oder Phospholipid-Lösung q.s. auf 100 ml umfaßt.

16. Substanzzusammensetzung nach einem der Ansprüche 1 bis 15, wenn sie in Einheitsdosierungsform vorliegt.

17. Substanzzusammensetzung nach Anspruch 16, wobei die Einheitsdosierungsform eine mit Flüssigkeit gefüllte Kapsel ist.

18. Verfahren zur Herstellung einer Substanzzusammensetzung, welches umfaßt: Lösen von Rapamycin in einer oder mehreren Komponenten eines Lösungsmittelsystems, das 0,05 bis 10 Vol.% Surfactant, 0 bis 25 Vol.% inertes Lösungsmittel und 65 bis 99,95 Vol.% Phospholipid-Lösung umfaßt, worin das Phospholipid in der Lösung 40 Masse-% bis 70 Masse-% ausmacht, so daß die Rapamycin-Konzentration in der gesamten Zusammensetzung 0,01 bis 10,0 g pro 100 ml beträgt.

19. Verfahren zur Herstellung einer Substanzzusammensetzung nach Anspruch 16, welches umfaßt: Lösen von Rapamycin in einer oder mehreren Komponenten eines Lösungsmittelsystems, das 0,1 bis 10 Vol.% Surfactant, 0,1 bis 25 Vol.% inertes Lösungsmittel und 65 bis 99,8 Vol.% einer Phospholipid-Lösung umfaßt, worin das Phospholipid in der Lösung 40 bis 60 Masse-% ausmacht, so daß die Rapamycin-Konzentration in der gesamten Zusammensetzung 0,01 bis 10,0 g pro 100 ml beträgt.

20. Verfahren zur Herstellung einer Substanzzusammensetzung nach Anspruch 16, welches umfaßt: Lösen von Rapamycin in einer oder mehreren Komponenten eines Lösungsmittelsystems, das 0,05 Vol.% bis 10 Vol.% Surfactant und 90 bis 99,95 Vol.% einer Phospholipid-Lösung umfaßt, worin das Phospholipid in der Lösung 40 bis 60 Masse-% ausmacht, so daß die Rapamycin-Konzentration in der gesamten Zusammensetzung 0,01 bis 1,0 g pro 100 ml beträgt.

## Revendications

1. Composition de matière contenant de 0,01 g à 10,0 g de rapamycine par 100 ml de composition et un système de solvant, ledit système de solvant comprenant de 0,05 à 10% en volume de surfactant, de 0 à 25% en volume de solvant inerte et de 65 à 99,95% en volume de solution de phospholipide, dans laquelle le phospholipide dans ladite solution est de 40 à 75% en poids.

2. Composition de matière suivant la revendication 1, contenant de 0,01 à 10,0 g de rapamycine par 100 ml de composition et un système de solvant, ledit système de solvant comprenant de 0,1 à 10% en volume de surfactant, de 0,1 à 25% en volume de solvant inerte et de 65 à 99,8% en volume d'une solution de phospholipide, dans laquelle le phospholipide dans ladite solution est de 40 à 60% en poids.

3. Composition de matière suivant la revendication 2, contenant de 0,05 à 5,0 g de rapamycine par 100 ml de composition et un système de solvant, comprenant de 0,5 à 8,0% en volume de surfactant, de 0,5 à 20% en volume de solvant inerte et de 72 à 99,0% en volume de solution de phospholipide.

4. Composition de matière suivant la revendication 2, contenant de 0,1 à 1,0 g de rapamycine par 100 ml de composition et un système de solvant comprenant de 1,0 à 5,0% en volume de surfactant, de 1,0 à 10% en volume de solvant inerte et de 85 à 98,0% en volume de solution de phospholipide.

5. Composition de matière suivant la revendication 1, contenant de 0,01 g à 1,0 g de rapamycine par 100 ml de composition et un système de solvant, ledit système de solvant comprenant de 0,05 à 10% en volume de surfactant et de 90% à 99,95% en volume de solution de phospholipide, le phospholipide dans ladite solution étant de 40 à 60% en poids.

6. Composition de matière suivant la revendication 5, contenant de 0,03 g à 0,8 g de rapamycine par 100 ml de composition et un système de solvant, ledit système de solvant comprenant de 0,10 à 5% en volume de surfactant et de 95% à 99,9% en volume d'une solution de phospholipide, le phospholipide dans ladite solution étant de 40 à 60% en poids.

7. Composition de matière suivant la revendication 6, contenant de 0,05 g à 0,5 g de rapamycine par 100 ml de composition et un système de solvant, ledit système de solvant comprenant de 0,5% à 5% en volume de surfactant et de 95% à 98,5% en volume d'une solution de phospholipide, le phospholipide dans ladite solution étant de 40 à 60% en poids.

8. Composition de matière suivant la revendication 5, contenant environ 1,0 g de rapamycine dans un système de solvant comprenant environ 1% en volume de surfactant et environ 99% en volume d'une solution de phospholipide contenant 50% en poids de phospholipide.

9. Composition de matière suivant l'une quelconque des revendications 1 à 7, dans laquelle le phospholipide constitue environ 50% en poids de la solution de phospholipide.

10. Composition de matière suivant l'une quelconque des revendications 1 à 9, dans laquelle le phospholipide est la lécithine.

11. Composition de matière suivant l'une quelconque des revendications 1 à 10, dans laquelle le surfactant est sélectionné parmi l'un des suivants : les esters de polyoxyéthylènesorbitol, les acides gras polyoxyéthylés, les alcools gras polyoxyéthylés et les esters gras hydroxylés de glycérol polyoxyéthylé.

12. Composition de matière suivant l'une quelconque des revendications 1 à 11, dans laquelle le solvant inerte est sélectionné, s'il est présent, parmi un ou plusieurs du diméthylacétamide, de l'éthanol, du glycérol, du diméthylformamide, du t-butanol, du polyéthylèneglycol et du propylèneqlycol.

13. Composition de matière suivant l'une quelconque des revendications 1 à 12, dans laquelle le solvant de la solution de phospholipide est le propylèneglycol.

14. Composition de matière comprenant, pour 100 ml de la composition :
a) un premier composant de 20 ml de 2500 mg de rapamycine dans le N,N-diméthylacétamide, et
b) un deuxième composant comprenant de 0,05 g/ml à 0,07 g/ml de surfactant dans de la lécithine, le deuxième composant étant ajouté au premier composant de 20 ml pour compléter à un volume de composition de 100 ml.

15. Composition de matière comprenant, pour 100 ml de composition, 2,5 g de rapamycine, 5,0 ml de surfactant, environ 13 ml d'éthanol absolu, et une solution à 50% de lécithine ou de phospholipide Q.S. pour 100 ml.

16. Composition de matière suivant l'une quelconque des revendications 1 à 15 sous la forme de dose unitaire.

17. Composition de matière suivant la revendication 16, dans laquelle la forme de dose unitaire est une capsule remplie de liquide.

18. Procédé de préparation d'une composition de matière, comprenant la dissolution de rapamycine dans un ou plusieurs composants d'un système de solvant comprenant de 0,05 à 10% en volume de surfactant, de 0 à 25% en volume de solvant inerte et de 65 à 99,95% en volume de solution de phospholipide, dans laquelle le phospholipide dans ladite solution est de 40 à 75% en poids, de telle sorte que la concentration en rapamycine dans la composition totale soit de 0,01 à 10,0 g par 100 ml.

19. Procédé de préparation d'une composition de matière suivant la revendication 16, comprenant la dissolution de rapamycine dans un ou plusieurs composants d'un système de solvant comprenant de 0,1 à 10% en volume de surfactant, de 0,1 à 25% en volume de solvant inerte et de 65 à 99,8% en volume d'une solution de phospholipide, dans laquelle le phospholipide dans ladite solution est de 40 à 60% en poids, de telle sorte que la concentration en rapamycine dans la composition totale soit de 0,01 à 10,0 g par 100 ml.

20. Procédé de préparation d'une composition de matière suivant la revendication 16, comprenant la dissolution de rapamycine dans un ou plusieurs composants d'un système de solvant comprenant de 0,05 à 10% en volume de surfactant et de 90% à 99,95% en volume d'une solution de phospholipide, le phospholipide dans ladite solution étant de 40 à 60% en poids, de telle sorte que la concentration en rapamycine dans la composition totale est de 0,01 à 1,0 g par 100 ml.
